# EUROPÄISCHE PATENTSCHRIFT

(11) **EP 0 789 012 B1**
(45) Veröffentlichungstag und Bekanntmachung des Hinweises auf die Patenterteilung: **26.04.2000**
(21) Anmeldenummer: 96120045.8
(22) Anmeldetag: 13.12.1996
(51) Int. Cl.: C07C 17/08, C07C 19/01

(54) **Verfahren zur Herstellung von Alkylhalogeniden**
Process for the preparation of alkyl halides
Procédé pour la préparation d'halogénures d'alkyle

(30) Priorität: 08.02.1996 DE 19604568
(43) Veröffentlichungstag der Anmeldung: 13.08.1997
(73) Patentinhaber: Degussa-Hüls Aktiengesellschaft, 60287 Frankfurt am Main (DE)
(72) Erfinder: Metz, Josef, Dr., 45770 Marl (DE); Osterholt, Clemens, 46286 Dorsten (DE)

(56) Entgegenhaltungen:
- BE-A- 669 733
- GB-A- 665 809

## Beschreibung

Die Erfindung betrifft ein neues Verfahren zur kontinuierlichen Herstellung von Alkylhalogeniden aus verzweigten Olefinen mit 4 bis 16 C-Atomen und konzentrierter Halogenwasserstoffsäure.

Alkylhalogenide dienen als Lösemittel. Sie werden darüber hinaus für Friedel-Crafts-Alkylierungen, zur Herstellung metallorganischer Verbindungen und zur Synthese von Pflanzenschutzmitteln und pharmazeutischen Produkten verwendet.

Bei der Herstellung der Alkylhalogenide geht man im allgemeinen von Alkoholen oder von Olefinen aus.

Nach US 3 852 368 erfolgt die Herstellung von tert.-Alkylchloriden in Lösemitteln wie Pentan, Hexan, Tetrachlorkohlenstoff oder Benzol in Gegenwart von tertiären Aminen in wäßriger Salzsäure bei tiefen Temperaturen.

US 2 434 092 beschreibt die Herstellung in Gegenwart von BF₃ bzw. BF₃-Addukten als Katalysatoren.

GB-A-665 809 and BE-A-669 733 beschreiben Verfahren zur Herstellung von Alkylhalogeniden in Gegenwart von nicht wässrigen Chlorwasserstoff.

Die genannten Verfahren sind umständlich bzw. aufwendig, weil Lösemittel abgetrennt und Katalysatoren aufgearbeitet werden müssen.

Aufgabe der vorliegenden Erfindung war es daher, ein vereinfachtes Verfahren zur Herstellung von Alkylhalogeniden aus Olefinen bereitzustellen.

Die Aufgabe wird erfindungsgemäß dadurch gelöst, daß man die Reaktion ohne Katalysatorzusatz und ohne Lösemittel durchführt.

Geeignete verzweigte Olefine sind beispielsweise Isobuten, 2-Methylbuten-2, 3-Methylpenten-2, 2,3-Dimethylbuten-2, 2,3-Dimethylpenten-2, 2-Ethylhexen-2, 2-Methylocten-1, 2,4-Dimethyldecen-1 oder Isopren. Vorzugsweise werden Olefine mit 4 bis 8 C-Atomen eingesetzt.

Als Halogenwasserstoffsäuren kommen Chlor-, Brom- und Jodwasserstoffsäure in Betracht. Konzentrierte Halogenwasserstoffsäuren im Sinne dieser Erfindungen sind mindestens 20%ige Halogenwasserstoffsäuren. Konzentrierte Salzsäuren sind 20- bis 45%ig.

Vorzugsweise werden nach dem erfindungsgemäßen Verfahren tertiäre Alkylchloride hergestellt.

Die Reaktionstemperatur ist unkritisch. Sie liegt im allgemeinen im Bereich von 0 bis 200 °C. Unterhalb des Bereichs ist die Reaktion langsam und z. T. unvollständig. Oberhalb von 200 °C treten verstärkt Verfärbungen des Produkts auf. Die Reaktionstemperatur liegt vorzugsweise im Bereich von 20 bis 160 °C.

Führt man die kontinuierliche Reaktion bei einer Temperatur oberhalb des Siedepunktes des Alkylhalogenids durch, so ist der sicherheitstechnische Aufwand vergleichweise gering. Dieses ist beispielsweise der Fall, wenn man tert.-Butylchlorid mit einem Siedepunkt von 51 °C bei einer Reaktionstemperatur von 60 °C herstellt.

Ein allgemeiner Vorteil des Verfahrens ist, daß die Reaktion bei Normaldruck durchgeführt werden kann. Überraschenderweise werden nach diesem Verfahren Alkylhalogenide bei hohen Olefinumsätzen, in hoher Reinheit und Selektivität und mit sehr guter Farbe erhalten. Deshalb sind keine oder nur sehr einfache destillative Reinigungsoperationen zur weiteren Farbverbesserung erforderlich. Außerdem ist kein Katalysatorhandling erforderlich.

Die folgenden Beispiele sollen die Erfindung erläutern.

### Beispiel 1

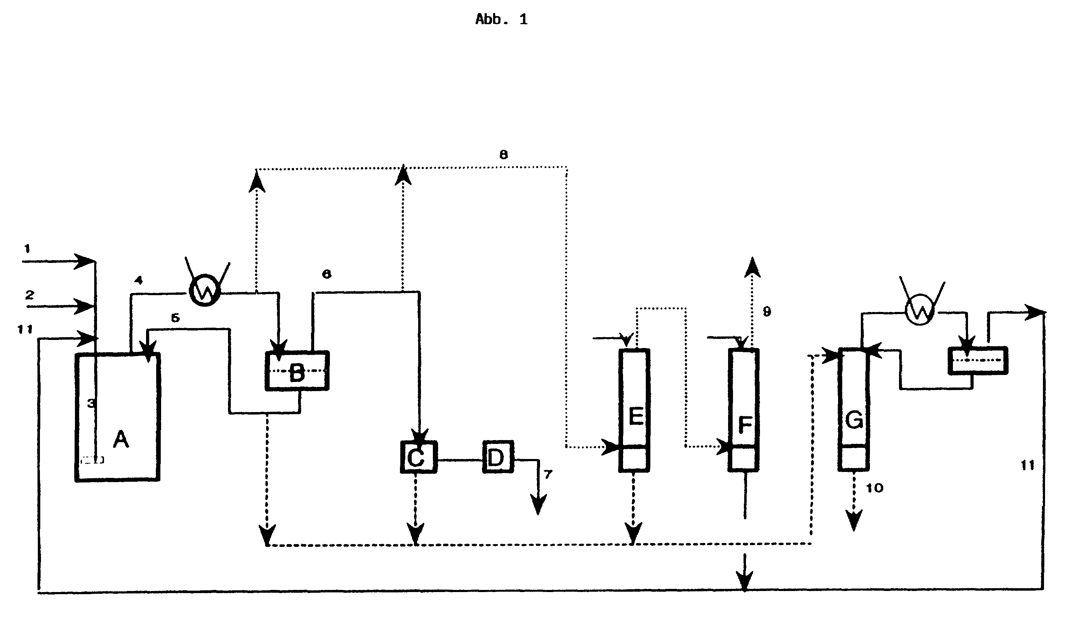

### Kontinuierliche Herstellung von tert.-Butylchlorid (Abb. 1)

In einem mit N₂ abgedeckten Glasreaktor (A) wird 1,5 kg konzentrierte Salzsäure (ca. 36%ig) vorgelegt, auf 60 °C eingestellt und über ein Tauchrohr (3), welches zur besseren Gasverteilung am unteren Ende mit einer Glasfritte ausgestattet ist, stündlich ein Gemisch aus 1 Mol Isobuten (1) und ca. 1,2 Mol Chlorwasserstoff (2) eingeleitet.

Das dabei anfallende Reaktionsprodukt wird dampfförmig abgezogen (4), kondensiert und das zweiphasige Destillat in der Vorlage (B) getrennt.

Die untere wäßrige Phase wird rückgeführt (5) und die obere Alkylchloridphase (6) zur Aufarbeitung [alkalische Wäsche (C) und alkalische Trocknung (D)] abgefahren.

Das so hergestellte tert.-Butylchlorid (7) wird mit einer Ausbeute von > 95 % der Theorie erhalten. Die Reinheit nach GC-Analytik beträgt ≥ 99,8 %; APHA-Farbzahl ≤ 10; H₂O-Gehalt ≤ 300 ppm.

Das Abgas, überwiegend Chlorwasserstoff und Stickstoff mit wenig Isobuten und tert.-Butylchlorid, wird über die Sammelleitung (8) zur Absorption von HCl dem H₂O-Gegenstromwascher (E) und anschließend zur Absorption von Alkylchloriden dem tert.-Butanol-Gegenstromwascher (F) zugeführt.

Das so gereinigte Abgas (9) kann einer Entsorgung, z. B. einer Abgasverbrennung, zugeführt werden.

Das mit tert.-Butylchlorid angereicherte tert.-Butanol aus dem Gegenstromwascher (F) wird in den Reaktor (A) eingespeist.

Die Prozeßabwässer werden auf einen pH-Wert von < 7 eingestellt und zur Entfernung von organischen Bestandteilen der Stripperkolonne (G) zugeführt. Die obere organische. Phase des dort anfallenden Azeotrops (überwiegend Olefine, Alkylchloride, Alkohole und wäßrige Salzsäure) wird in den Reaktor (A) zurückgeführt und die untere wäßrige Azeotropphase in den oberen Teil der Stripperkolonne gefahren.

Der von organischen Anteilen befreite Sumpf (10) wird einer Abwasseraufbereitungsanlage zugeführt.

### Beispiel 2

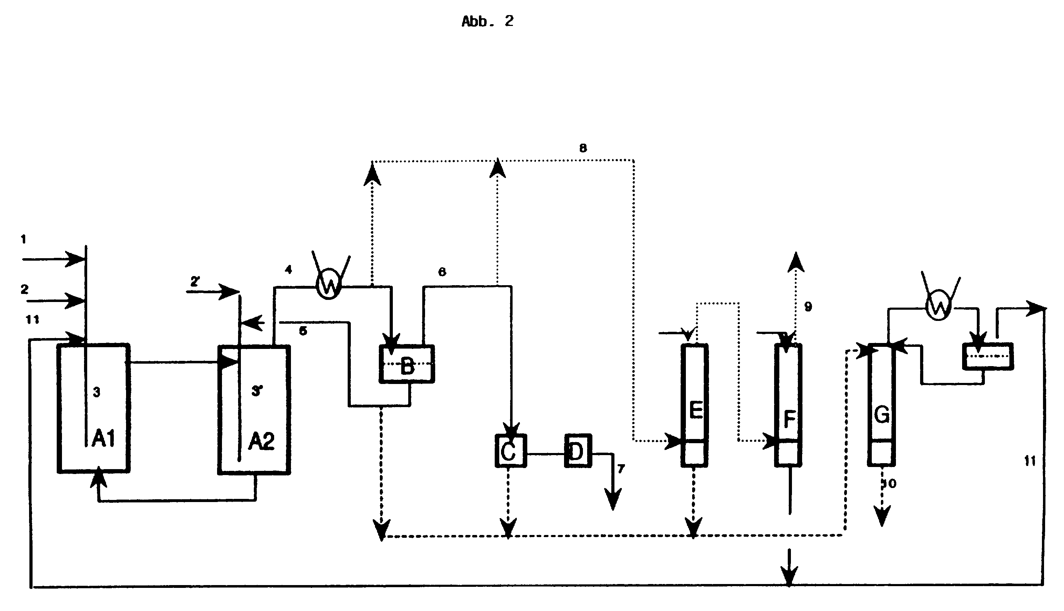

### Kontinuierliche Herstellung von tert.-Amylchlorid (Abb. 2)

Zwei in Reihe geschaltete Glasreaktoren (A₁, A₂) werden jeweils mit ca. 2 kg konzentrierter Salzsäure (ca. 36%ig) gefüllt. Der Reaktor A₁ wird auf 30 °C und der Reaktor A₂ auf 90 °C eingestellt.

In den Reaktor A₁ werden über das Tauchrohr (3) stündlich ein Gemisch aus ca. 1 Mol 2-Methylbuten-2 (97,7%ig) und ca. 1 Mol Chlorwasserstoff eingeleitet.

Das Reaktionsprodukt gelangt per Überlauf in den Reaktor A₂. Dort werden zusätzlich 0,3 Mol Chlorwasserstoff (2') zudosiert, und das Reaktionsprodukt wird über Kopf (4) abgezogen.

Das kondensierte, zweiphasige Destillat wird in der Vorlage B getrennt.

Die weitere Aufarbeitung erfolgt analog zu Beispiel 1. Der Gegenstromwascher (F) wird mit 2-Methylbutanol-2 betrieben.

Bei dieser Kaskadenfahrweise beträgt der Olefinumsatz im ersten Reaktor > 95 %, die Restumsetzung erfolgt in der zweiten Reaktionsstufe.

Das so hergestellte tert.-Amylchlorid (7) wird mit einer Ausbeute von > 95 % der Theorie erhalten. Die Reinheit nach GC-Analytik beträgt 98,0 %; APHA-Farbzahl ≤ 10; H₂O-Gehalt ≤ 200 ppm.

### Beispiel 3

### Kontinuierliche Herstellung von tert.-Hexylchlorid (Abb. 2)

Zwei in Reihe geschaltete Glasreaktoren (A₁, A₂) werden jeweils mit ca. 2 kg konzentrierter Salzsäure (ca. 36%ig) befüllt. Der Reaktor A₁ wird auf 40 °C und der Reaktor A₂ auf 105 °C eingestellt.

In den Reaktor A₁ werden über das Tauchrohr (3) stündlich ein Gemisch aus ca. 1 Mol 3-Methylpenten-2 (cis/trans-Gehalt 88 %, 2-Ethylbuten-1 = 12 %) und ca. 1 Mol Chlorwasserstoff (2) eingeleitet.

Das Reaktionsprodukt gelangt per Überlauf in den Reaktor A₂. Dort werden zusätzlich 0,3 Mol Chlorwasserstoff (2') zudosiert, und das Reaktionsprodukt wird als Azeotrop über Kopf (4) abgezogen.

Das kondensierte, zweiphasige Destillat wird in der Vorlage B getrennt.

Die weitere Aufarbeitung erfolgt analog zu Beispiel 1. Der Gegenstromwascher (F) wird mit 3-Methylpentanol-3 betrieben.

Bei dieser Kaskadenfahrweise beträgt der Olefinumsatz im ersten Reaktor ca. 93 %, die Restumsetzung erfolgt in der zweiten Reaktionsstufe.

Das so hergestellte tert.-Hexylchlorid (7) wird mit einer Ausbeute von > 95 % der Theorie erhalten. Die Reinheit nach GC-Analytik beträgt 98,7 %; APHA-Farbzahl 30; H₂O-Gehalt ≤ 200 ppm.

## Patentansprüche

1. Verfahren zur kontinuierlichen Herstellung von Alkylhalogeniden aus verzweigten Olefinen mit 4 bis 16 C-Atomen und mindestens 20- bis 45%iger Halogenwasserstoffsäure,
dadurch gekennzeichnet,
daß man die Reaktion ohne Katalysatorzusatz und ohne Lösemittel durchführt.

2. Verfahren nach Anspruch 1,
dadurch gekennzeichnet,
daß die Olefine 4 bis 8 C-Atome aufweisen.

3. Verfahren nach Anspruch 1,
dadurch gekennzeichnet,
daß die Reaktionstemperatur bei 20 bis 160 °C liegt.

4. Verfahren nach Anspruch 1,
dadurch gekennzeichnet,
daß tertiäre Alkylchloride hergestellt werden.

## Claims

1. A process for the continuous preparation of alkyl halides from branched olefins having 4 to 16 carbon atoms and at least 20- to 45% strength hydrohalic acid, characterized in that the reaction is carried out without the addition of catalyst and without solvents.

2. A process according to claim 1, characterized in that the olefins have 4 to 8 carbon atoms.

3. A process according to claim 1, characterized in that the reaction temperature is from 20 to 160°C.

4. A process according to claim 1, characterized in that tertiary alkyl chlorides are prepared.

## Revendications

1. Procédé de fabrication en continu d'halogénures d'alkyle à partir d'oléfines ramifiées ayant de 4 à 16 atomes de C, et d'acide halohydrique d'au moins 20 % jusqu'à 45 %,
caractérisé en ce qu'
on effectue la réaction sans ajout de catalyseur et sans agent dissolvant;

2. Procédé selon la revendication 1,
caractérisé en ce que
les oléfines possèdent de 4 à 8 atomes de carbone.

3. Procédé selon la revendication 1,
caractérisé en ce que
la température de réaction se situe de 20 à 160°C.

4. Procédé selon la revendication 1,
caractérisé en ce que
des chlorures d'alkyle tertiaires sont produits.
